# EUROPEAN PATENT APPLICATION

(11) **EP 4 574 263 A1**
(43) Date of publication of application: **25.06.2025**
(21) Application number: 24218796.1
(22) Date of filing: 10.12.2024
(51) Int. Cl.: B01L 9/00

(54) **FILTER SUCTION HOLDER, NUCLEIC ACID EXTRACTION SYSTEM, AND NUCLEIC ACID EXTRACTION METHOD**

(30) Priority: 22.12.2023 JP 2023217112
(71) Applicant: Yokogawa Electric Corporation, Tokyo 180-8750 (JP)
(72) Inventor: MITSUMORI, Yuuji, Musashino-shi, Tokyo, 180-8750 (JP); TAGUCHI, Tomoyuki, Musashino-shi, Tokyo, 180-8750 (JP)
(74) Representative: Zimmermann, Tankred Klaus

(57) **Abstract**

A filter suction holder 310 is insertable into a reaction tube together with a filter. The filter is configured to capture a specimen having nucleic acid by filtering a sample containing the specimen. The reaction tube is to be used for extracting the nucleic acid from the specimen. The filter suction holder 310 includes a container 311 for containing the filter. The container 311 has an inner wall surface 312 configured to stick to a surface of the filter and cause the filter to be absorbed.

## Description

### CROSS-REFERENCE TO RELATED APPLICATION

This application claims priority to Japanese Patent Application No. 2023-217112 filed on December 22, 2023, the entire contents of which are incorporated herein by reference.

### TECHNICAL FIELD

The present disclosure relates to a filter suction holder, a nucleic acid extraction system, and a nucleic acid extraction method.

### BACKGROUND

Patent Literatures (PTLs) 1 and 2 disclose nucleic acid extraction methods.

In the conventional nucleic acid extraction methods, microorganisms in a sample are recovered on a surface of a membrane filter by filtering the sample through the membrane filter. The membrane filter is then picked up with sterilized tweezers, and inserted into a reaction tube so that the membrane filter is contained in the reaction tube. Thereafter, nucleic acid is extracted from the microorganisms by adding a surface-active agent into the reaction tube and heating the reaction tube to a temperature of a boiling point or more to make a high-temperature and high-pressure state.

### CITATION LIST

### Patent Literatures

PTL 1: WO 2019/043779 A1
PTL 2: JP 2012-157265 A

### SUMMARY

A process of collecting a filter after filtration and inserting the collected filter into a reaction tube is very complicated because the extremely thin filter has to be handled with tweezers. Furthermore, in order to extract nucleic acid, the filter needs to be immersed in a reaction solution containing a surface-active agent, but it is difficult to insert the filter into the bottom of the reaction tube because the wet filter easily sticks to an inner wall of the reaction tube.

It would be helpful to make it easier to insert a filter into a reaction tube.

A filter suction holder, a nucleic acid extraction system, and a nucleic acid extraction method according to some embodiments will be described below.
[1] A filter suction holder insertable into a reaction tube together with a filter, the filter being configured to capture a specimen having nucleic acid by filtering a sample containing the specimen, the reaction tube to be used for extracting the nucleic acid from the specimen, the filter suction holder including:
   a container for containing the filter, the container having an inner wall surface configured to stick to a surface of the filter and cause the filter to be absorbed.
   Using such a filter suction holder makes it easier to insert the filter into the reaction tube.
[2] The filter suction holder according to [1], wherein
   the container is in shape of a hollow cone or a hollow truncated cone,
   the cone or the truncated cone is open at a bottom to expose the inner wall surface, and
   the container is configured to be inserted into the reaction tube from an end of the cone or the truncated cone with a smaller diameter.
   Using such a filter suction holder makes it easier to insert the filter into the bottom of the reaction tube.
[3] The filter suction holder according to [1] or [2], wherein the container has a heat resistance of at least 180°C.
   In such a filter suction holder, it is possible to prevent unintended deterioration during heating.
[4] The filter suction holder according to any one of [1] to [3], wherein the container is formed of a material that melts when a certain amount of heat is applied.
   With the use of such a filter suction holder, the filter suction holder melts during extraction of the nucleic acid and increases the area of contact between the filter and a reaction solution containing a surface-active agent, thus improving reaction efficiency.
[5] The filter suction holder according to any one of [1] to [4], further including a surface-active agent fixed to the container.
   Using such a filter suction holder can simplify operation of adding the surface-active agent, which is required for extraction of the nucleic acid, to the reaction tube.
[6] A nucleic acid extraction system including:
   the filter suction holder according to any one of [1] to [5]; and
   a reaction tube capable of containing the filter suction holder together with the filter.
   In such a nucleic acid extraction system, it becomes easier to insert the filter into the reaction tube.
[7] A nucleic acid extraction method including:
   capturing, using a filter, a specimen having nucleic acid by filtering a sample containing the specimen;
   containing the filter in a container of a filter suction holder, the container having an inner wall surface configured to stick to a surface of the filter and cause the filter to be absorbed;
   inserting the filter suction holder into a reaction tube together with the filter; and
   extracting, using the reaction tube, the nucleic acid from the specimen.

In such a nucleic acid extraction method, it becomes easier to insert the filter into the reaction tube.

The present disclosure can make it easier to insert a filter into a reaction tube.

### BRIEF DESCRIPTION OF THE DRAWINGS

In the accompanying drawings:
FIG. 1 is a diagram illustrating a configuration of a nucleic acid analysis system according to an embodiment of the present disclosure;
FIG. 2 is a diagram illustrating an example of a filter and tweezers according to the embodiment of the present disclosure;
FIG. 3 is a diagram illustrating an example of a filter suction holder according to the embodiment of the present disclosure;
FIG. 4 is a diagram illustrating an example of reaction tubes according to the embodiment of the present disclosure; and
FIG. 5 is a flowchart illustrating a nucleic acid extraction method according to the embodiment of the present disclosure.

### DETAILED DESCRIPTION

An embodiment of the present disclosure will be described below with reference to the drawings.

In the drawings, the same or equivalent parts are indicated with the same reference numerals. In descriptions of each embodiment, descriptions of the same or equivalent parts are omitted or simplified as appropriate.

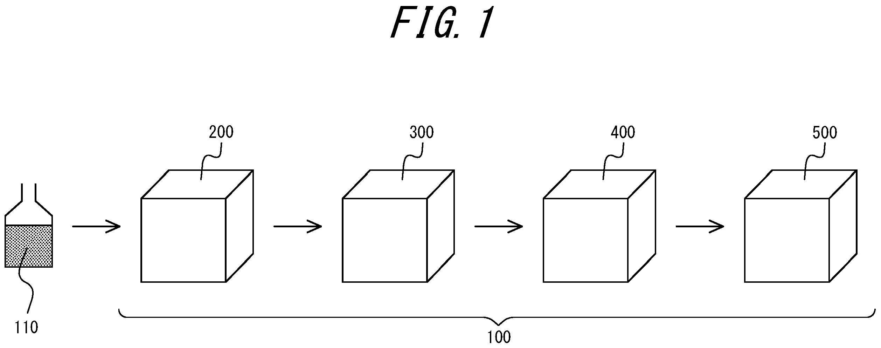

A configuration of a nucleic acid analysis system 100 according to the present embodiment will be described with reference to FIG. 1.

The nucleic acid analysis system 100 includes a specimen recovery system 200, a nucleic acid extraction system 300, a hybridization reaction system 400, and a detection system 500.

The specimen recovery system 200 is a system that recovers, from a sample 110, a specimen contained in the sample 110.

The specimen is, for example, bacteria, fungi, viruses, or other microorganisms. When a test is carried out on a beverage, the sample 110 may be the beverage manufactured. Alternatively, the sample 110 may be water to be used for manufacturing the beverage. Alternatively, the sample 110 may be liquid produced in the process of manufacturing the beverage. When bacteria are recovered into a liquid from a swab with which testing environment has been wiped, by vibrating the swab in the liquid or vibrating the liquid containing the swab, in order to test the presence or absence of contamination by bacteria in manufacturing environment or the degree of the contamination, the sample 110 may be that liquid.

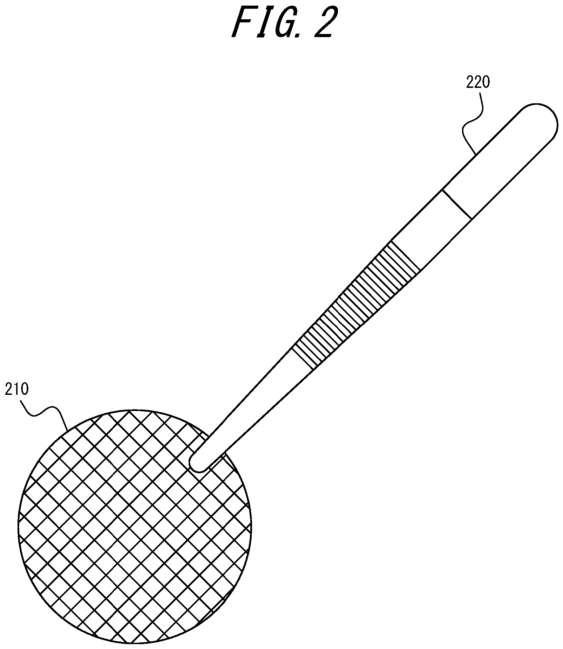

The specimen recovery system 200 includes a filter 210 as illustrated in FIG. 2.

The recovery is carried out by, for example, pressurizing or depressurizing the sample 110 and filtering the sample 110 through the filter 210. When bacteria or fungi are recovered, the pore size of the filter 210 is, for example, 0.45 mm or 0.22 mm.

Specifically, the filter 210 is a membrane filter. The diameter of the filter 210 is, for example, 13 mm. The filter 210 is required to be a size insertable into a reaction tube 320, which is described below. The shape of the filter 210 is, for example, a circle. The material of the filter 210 is, for example, MCE, PVDF, PES, or PC. "MCE" is an abbreviation for mixed cellulose ester. "PVDF" is an abbreviation for polyvinylidene fluoride. "PES" is an abbreviation for polyethersulfone. "PC" is an abbreviation for polycarbonate.

The nucleic acid extraction system 300 is a system that extracts, from the specimen recovered by the specimen recovery system 200, nucleic acid contained in the specimen.

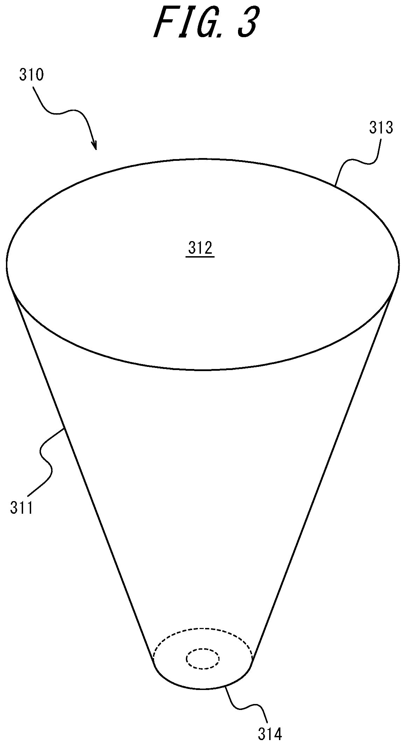

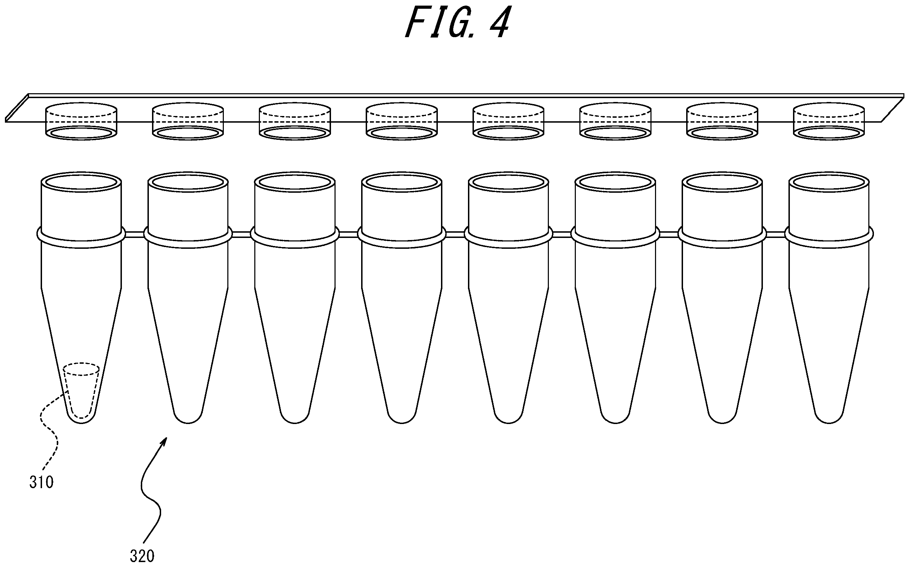

The nucleic acid extraction system 300 includes a filter suction holder 310 as illustrated in FIG. 3, and a reaction tube 320 as illustrated in FIG. 4.

After the specimen is recovered by the filter 210, the filter 210 is transferred to the filter suction holder 310 using tweezers 220 as illustrated in FIG. 2. The filter suction holder 310 adsorbs the filter 210. The filter suction holder 310 is inserted into the reaction tube 320 together with the filter 210. In the present embodiment, the filter 210 is not directly inserted into the reaction tube 320 using the tweezers 220, but is contained in the filter suction holder 310 and then inserted into the reaction tube 320. Therefore, the filter suction holder 310 can prevent the filter 210 from falling out. Using the filter suction holder 310 allows the filter 210 to be fixed in place, making it easier to insert the filter 210 into the reaction tube 320. Using the filter suction holder 310 prevents the filter 210 from sticking to an inner wall of the reaction tube 320, making it easier to insert the filter 210 into the bottom of the reaction tube 320. Since the filter suction holder 310 is disposable, the risk of contamination can also be reduced.

Vacuum tweezers may be used instead of the tweezers 220. Using the vacuum tweezers eliminates the need for picking up the filter 210 with the tweezers 220, and the filter 210 can be collected with simple operation. Therefore, the filter 210 can be reliably collected without being affected by a skill of a worker.

The filter suction holder 310 is inserted into the reaction tube 320 together with the filter 210, after the filter 210 has captured the specimen by filtering the sample 110. Therefore, the filter suction holder 310 is in such a size as to be insertable into the reaction tube 320.

The filter suction holder 310 has a container 311 to contain the filter 210. The container 311 has an inner wall surface 312 that sticks to a surface of the filter 210 and causes the filter 210 to be adsorbed.

In the present embodiment, the container 311 is in the shape of a hollow cone or hollow truncated cone. The bottom of the cone or truncated cone is open so as to expose the inner wall surface 312. The container 311 has a first end 313 corresponding to the bottom of the cone or truncated cone, and a second end 314 corresponding to the apex of the cone or the opposite bottom of the truncated cone. The container 311 is inserted into the reaction tube 320 from the second end 314, which has a smaller diameter, of the cone or truncated cone, out of the first end 313 and the second end 314.

In the present embodiment, the container 311 is open not only at the bottom of the cone or truncated cone, but also at the apex of the cone or the opposite bottom of the truncated cone. In other words, the container 311 has openings at both the first end 313 and the second end 314.

The container 311 preferably has a heat resistance of at least 180°C. For example, the container 311 may be formed of PES.

The container 311 may be formed of a material that melts when a certain amount of heat is applied. For example, the container 311 may be formed of a thermally degradable polymer. Since melting the container 311 increases the area of contact between the filter 210 and a reaction solution containing a surface-active agent during extraction of the nucleic acid, an improvement in reaction efficiency can be expected.

The filter suction holder 310 may be further provided with a surface-active agent fixed to the container 311. For example, a reaction solution containing the surface-active agent used for extracting the nucleic acid may be dried in advance and fixed to the container 311, thereby simplifying operation of adding the reaction solution.

The reaction tube 320 has such a structure that can contain the filter suction holder 310 together with the filter 210. As the reaction tube 320, a general PCR tube can be used. "PCR" is an abbreviation for polymerase chain reaction. The capacity of the reaction tube 320 is, for example, 0.2 mL. A method of extracting, using the reaction tube 320, the nucleic acid from the specimen is the same as known one, such as a method disclosed in PTL 1 or 2, except that the filter suction holder 310 is used, so a description is omitted.

The sample 110 in which the nucleic acid has been extracted may be mixed with a liquid containing other nucleic acid B that reacts with the extracted nucleic acid. The nucleic acid B may be nucleic acid to which a part having a fluorescence, luminescence, or quenching action under specific conditions has been imparted for detection by the detection system 500.

The hybridization reaction system 400 is a system that causes a hybridization reaction in the sample 110.

In the hybridization reaction system 400, the sample 110 is heated to, for example, 60°C and agitated so that the reaction occurs between the above-described nucleic acid B and the matching nucleic acid. For example, since the part having the fluorescence, luminescence, or quenching action under the specific conditions, which has been imparted to the nucleic acid B, reacts with the nucleic acid in the sample 110, the fluorescence, luminescence, or quenching action is manifest. Using, as the nucleic acid B, nucleic acid that is structurally designed to react with specific nucleic acid allows the nucleic acid B to react only with the nucleic acid structure of specific microorganisms, for example. Therefore, using the specific nucleic acid B in this hybridization reaction system 400 makes it possible to cause the fluorescence, luminescence, or quenching action, which has been imparted to the nucleic acid B, to be manifest only when the specific microorganisms are present in the sample 110.

The detection system 500 is a system that detects the presence or absence of the fluorescence, luminescence, or quenching action that has been manifest by the reaction of the nucleic acid B, and the degree thereof, in the sample 110 processed by the hybridization reaction system 400.

For example, the detection may be carried out by exciting, using an excitation laser beam, the fluorescence action that has been imparted to the nucleic acid B and has been manifest by the reaction with the nucleic acid in the sample 110, and then detecting fluorescence after the excitation with a high-sensitivity camera. Alternatively, the detection may be carried out by detecting, with a high-sensitivity camera, the luminescence action that has been imparted to the nucleic acid B and has been manifest by the reaction with the nucleic acid in the sample 110. Alternatively, for the quenching action that has been imparted to the nucleic acid B and has been manifest by the reaction with the nucleic acid in the sample 110, the detection may be carried out by detecting, with a high-sensitivity camera, the degree of quenching fluorescence or luminescence imparted in the vicinity of the part to which the quenching action has been imparted.

Using such a nucleic acid analysis system 100 makes it possible to analyze whether the specific microorganisms are present in the sample 110, or the concentration of the specific microorganisms.

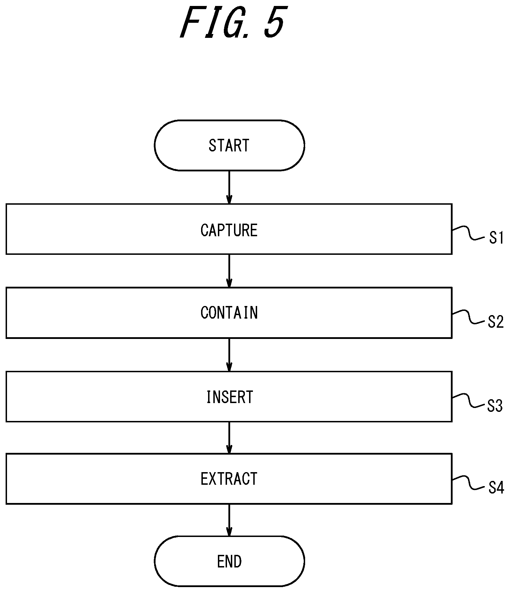

A nucleic acid extraction method according to the present embodiment will be described with reference to FIG. 5.

The nucleic acid extraction method according to the present embodiment includes a capture process S 1, a containment process S2, an insertion process S3, and an extraction process S4. The capture process S1 is a process of capturing a specimen by filtering, using a filter 210, a sample 110 containing the specimen having nucleic acid. The containment process S2 is a process of containing the filter 210 in a container 311, which has an inner wall surface 312 that sticks to a surface of the filter 210 and causes the filter 210 to be adsorbed, of a filter suction holder 310. The insertion process S3 is a process of inserting the filter suction holder 310 into a reaction tube 320 together with the filter 210. The extraction process S4 is a process of extracting, using the reaction tube 320, the nucleic acid from the specimen.

The use of such a nucleic acid extraction method makes it easier to insert the filter 210 into the reaction tube 320.

The present disclosure is not limited to the above-described embodiment. Various modifications may be implemented within the scope of not deviating from the gist of the present disclosure.

## Claims

1. A filter suction holder insertable into a reaction tube together with a filter, the filter being configured to capture a specimen having nucleic acid by filtering a sample containing the specimen, the reaction tube to be used for extracting the nucleic acid from the specimen, the filter suction holder comprising:
a container for containing the filter, the container having an inner wall surface configured to stick to a surface of the filter and cause the filter to be absorbed.

2. The filter suction holder according to claim 1, wherein
the container is in shape of a hollow cone or a hollow truncated cone,
the cone or the truncated cone is open at a bottom to expose the inner wall surface, and
the container is configured to be inserted into the reaction tube from an end of the cone or the truncated cone with a smaller diameter.

3. The filter suction holder according to claim 1, wherein the container has a heat resistance of at least 180°C.

4. The filter suction holder according to claim 1, wherein the container is formed of a material that melts when a certain amount of heat is applied.

5. The filter suction holder according to claim 1, further comprising a surface-active agent fixed to the container.

6. A nucleic acid extraction system comprising:
the filter suction holder according to any one of claims 1 to 5; and
a reaction tube capable of containing the filter suction holder together with the filter.

7. A nucleic acid extraction method comprising:
capturing, using a filter, a specimen having nucleic acid by filtering a sample containing the specimen;
containing the filter in a container of a filter suction holder, the container having an inner wall surface configured to stick to a surface of the filter and cause the filter to be absorbed;
inserting the filter suction holder into a reaction tube together with the filter; and
extracting, using the reaction tube, the nucleic acid from the specimen.
